# EUROPEAN PATENT APPLICATION

(11) **EP 4 506 679 A1**
(43) Date of publication of application: **12.02.2025**
(21) Application number: 23845306.2
(22) Date of filing: 10.07.2023
(51) Int. Cl.: G01N 21/31, G01N 21/78

(54) **METHOD FOR FITTING STANDARD CURVE FOR ANALYZING SUBSTRATE CONCENTRATION IN ENZYMATIC REACTION**

(30) Priority: 29.06.2023 CN 202310783298
(71) Applicant: Xu, Zhancheng, Shijiazhuang, Hebei 050011 (CN)
(72) Inventor: Xu, Zhancheng, Shijiazhuang, Hebei 050011 (CN)
(74) Representative: Bayramoglu et al.
(86) International application number: PCT/CN2023/106493
(87) International publication number: WO 2024/022093

(57) **Abstract**

A method of fitting the standard curve to analyze the substrate concentration by enzyme catalysis is provided, including the following steps: within the preset concentration range of substrate and the concentration range of its corresponding tool enzyme, select an enzyme at any concentration within the range to react with a substrate at any concentration within the range, test the optical signal, and obtain the standard curves for analyzing the substrate concentration by calculating with multiple reaction curves formed beforehand. By applying the method of this invention, the tedious steps of testing, analyzing and fitting the standard curve by enzyme catalysis by the prior art can be reduced, materials can be saved and the test efficiency can be improved without reducing the accuracy of the prior art.

## Description

### TECHNICAL FIELD

The present invention relates generally to the field of biochemistry photoanalysis, and specifically to a method of fitting the standard curve to analyze the substrate concentration by enzyme catalysis.

### BACKGROUND TECHNOLOGY

In the field of testing and examination, enzyme catalysis is widely applied. The test of substrates, such as in mono-enzyme analysis technology and enzyme coupling analysis technology, etc., all involves indicating the reaction degree by color developing or illumining of the reactants or the products. Among them, there are two types of indicator reactions commonly used in enzyme coupling analysis technology. One is using oxidoreductase reaction to connect it to the forward/reverse reaction of NAD(P)H, after which, the change of NAD(P)H is measured directly by spectrophotometry or other methods. The other is catalyzing and oxidizing the substrate by oxidase to produce hydrogen peroxide, after which, add peroxidase and chromogenic agent for color comparison. The chromogenic agents commonly used by peroxidase are mixture of 4-amino-antipyrine and phenols, tetramethyl benzidine, o-phenylenediamine and o-tolidine. Based on the principle that the color developing of reactants or products indicates the degree of reaction, the content of the substrate is analyzed by creating the standard curve that takes the reaction coloring values as the vertical coordinates and the substrate concentrations as the horizontal coordinates.

The substrate concentration is analyzed by the prior art with the principle that the substrate concentration is directly proportional to the reaction rate in the first-order reaction of enzyme catalysis. A tool enzyme at an appropriate concentration is used to react with the substrate at multiple different concentrations to test optical signals and a standard curve is created to analyze the substrate concentration. Generally, the vertical coordinates are 3-6 optical signals obtained by testing and the horizontal coordinates are 3-6 corresponding substrate concentrations.

The tool enzyme used to analyze the substrate concentration is bioactive substance. It will change over time even under stable storage conditions, so, to ensure accuracy, the creation of a standard curve is a necessity before each experiment. Before each experiment, the standard curve with about five points should be created. The operation is tedious and the reagents of tool enzyme, color developing and illumining are expensive. A method is urgently needed to reduce the tedious operation of creating traditional standard curves before each experiment, save costs, reduce the consumption of tool enzyme reagents and color developing reagents and improve test efficiency.

### CONTENT OF THE INVENTION

This invention provides a method of fitting the standard curve to analyze the substrate concentration by enzyme catalysis. By applying this method, on the premise of ensuring the accuracy of the test, the standard curve for analyzing the substrate concentration can be obtained by calculating with multiple preset reaction curves after reacting and testing only once. The consumption of the tool enzymes and color developing reagents is saved, the tedious operations of starting analytical equipment for each test or creating the standard curve by the prior art before the experiment is reduced and the mechanical structure of automatic test equipment is simplified.

### TECHNICAL PROPOSAL

A method of fitting the standard curve to analyze the substrate concentration by enzyme catalysis is characterized by the following steps: step 1, set a range of concentrations for analyzing the substrate; step 2, test the optical signals by enzyme catalysis, and determine a range of the suitable tool enzyme concentrations for testing the substrate concentration in the range of step 1; step 3, by the enzyme catalysis process of step 2, select enzyme at multiple different concentrations within the set range of the tool enzyme concentrations in step 2 to react with substrate at multiple different concentrations within the substrate concentration range, and fit at least three or more reaction curves and get the curve equations; step 4, use the reaction curve fitted in step 3 to test enzyme at any concentration within the range of the tool enzyme concentrations in step 2 to obtain an optical signal; step 5, substitute the optical signal obtained in step 4 into the reaction curve equation of the corresponding substrate in step 3 to obtain an enzyme concentration value; step 6, substitute the enzyme concentration value into the other reaction curve equations in step 3 and three or more optical signals are obtained by calculation; step 7, take three or more optical signals obtained in step 6 as the vertical coordinates and the corresponding substrate concentrations as the horizontal coordinates, create a curve and a curve equation, and obtain the standard curve of testing the substrate concentration by tool enzyme at any concentration within the range of tool enzyme concentrations in step 2.

The said optical signals refer to the visible light and the invisible light, which are specifically manifested as absorbance, reflection intensity, fluorescence intensity and chemiluminescence intensity.

The said enzyme catalysis is carried out under the same reaction system, which refers to setting the range of concentrations for analyzing the substrate and the range of the suitable tool enzyme concentrations for testing and analysis. The other conditions in the reaction system remain the same.

The said reaction curves fitted and curve equations generated in step 3 are the ones of testing the enzyme concentration by the prior art, that is, the vertical coordinates are 3-5 or more optical signals and the horizontal coordinates are 3-5 or more corresponding tool enzyme concentrations.

Based on the principle that the substrate concentration is directly proportional to the reaction rate in the first-order reaction of enzyme catalysis, the reaction system can be presented as follows.

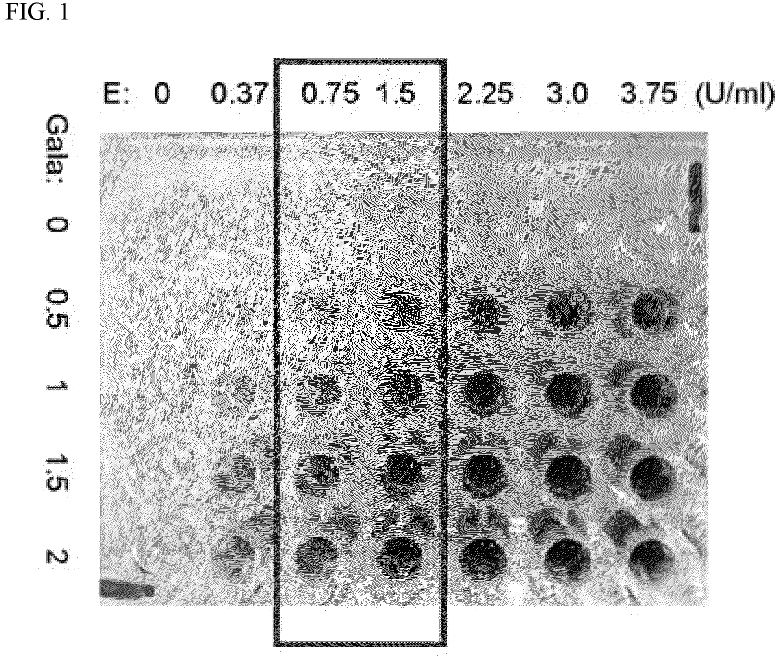

It can be observed that when the enzyme at different concentrations is used, such as 0.75 U/mL - 1.5 U/mL, color developing in the upper and lower of the substrate range can be achieved, and each reaction color can be found with its corresponding substrate concentration and enzyme concentration.

Select enzyme at multiple different concentrations within the set range of the tool enzyme concentrations and use it to react with substrate at multiple different concentrations within the substrate concentration range, and form beforehand three or more reaction curves and curve equations for analyzing the enzyme concentration. Any enzyme in the range of 0.75 U/mL - 1.5 U/mL is used to react with substrate at any concentration in the substrate concentration range. The enzyme concentration value can be calculated through the curve that uses the corresponding substrate to analyze the enzyme concentration. This enzyme concentration value is substituted into the multiple reaction curve equations formed beforehand for analyzing the enzyme concentration, and the optical signal values of this enzyme reacting with substrate at multiple different concentrations are obtained by calculation. The standard curve for analyzing the substrate concentration can be obtained by calculating with the obtained multiple optical signal values and the corresponding substrate concentrations.

The method in this invention only needs to react and test once and the standard curve for analyzing the substrate concentration can be obtained by calculation, which can save materials, reduce operation steps and is more efficient and faster than the method of creating the standard curve before the experiment by the prior art.

### SPECIFIC IMPLEMENTATIONS

The following embodiments are intended to deepen the understanding to this invention and do not represent the entire content of this invention which includes but is not limited to the following embodiments.

### Embodiment 1

The standard curve is fitted by using glucose oxidase to carry out Trinder reaction to analyze the glucose concentration. The range of concentrations for analyzing the substrate is set to be 0.06 - 0.12 mmol/L. The suitable test range of the enzyme concentrations obtained from the experiment is 3.0 - 6.5 U/mL. In the set range for analyzing substrate and its corresponding range of the enzyme concentrations of 3.0 - 6.5 U/mL, multiple points are selected to react and three or more reaction curves for testing the enzyme concentration by the prior art are formed beforehand.
The first curve, the curve of the substrate near the median value of the range reacting with multi-point enzyme within the suitable range;
The second curve, the curve of the substrate in the upper limit of the range reacting with multi-point enzyme in the suitable range;
The third curve, the curve of the substrate in the lower limit of the range reacting with multi-point enzyme within the suitable range.

X axis represents the concentration of glucose oxidase, Y axis represents the absorbance obtained, and the reaction time is 15 mins.

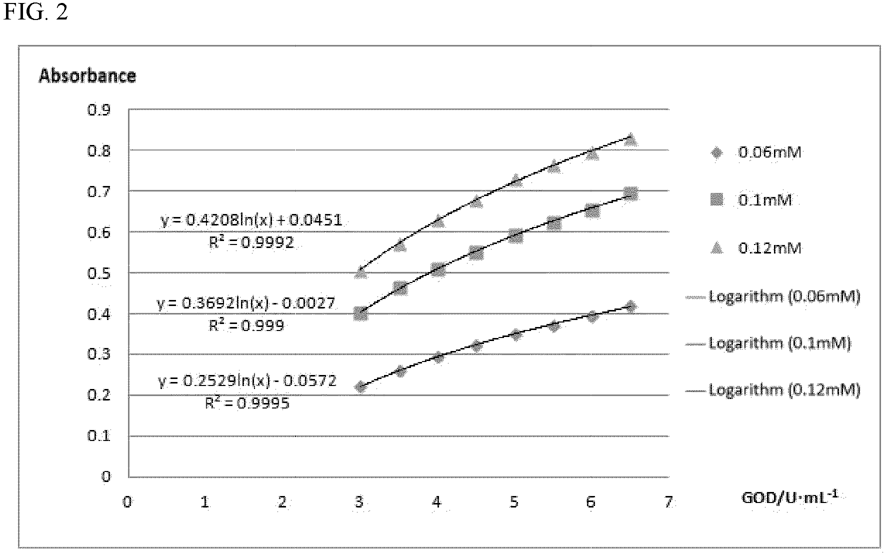

The upper curve is a curve of the glucose solution (0.12 mmol/L) in the upper limit of the range reacting with the enzyme at eight different concentrations (3.0 U/mL, 3.5 U/mL, 4.0 U/mL, 4.5 U/mL, 5.0 U/mL, 5.5 U/mL, 6.0 U/mL, 6.5 U/mL).

The middle curve is a curve of the glucose solution (0.1mmol /L) near the median value of substrate range reacting with the enzyme at eight different concentrations (3.0U /mL, 3.5U/mL, 4.0U /mL, 4.5U /mL, 5.0U /mL, 5.5 U/mL, 6.0U /mL, 6.5U /mL).

The lower curve is a curve of the glucose solution (0.06 mmol/L) in the lower limit of the range reacting with the enzyme at eight different concentrations (3.0 U/mL, 3.5 U/mL, 4.0 U/mL, 4.5 U/mL, 5.0 U/mL, 5.5 U/mL, 6.0 U/mL, 6.5 U/mL).

By using any enzyme (unknown) in the range to react with 0.1 mmol/L glucose solution, the absorbance value obtained from the reaction is substituted into the reaction curve equation of 0.1 mmol/L glucose solution and the enzyme concentration value is obtained. This enzyme concentration value is substituted into the reaction curve equation of 0.12 mmol/L glucose solution and the reaction absorbance value of 0.12 mmol/L glucose solution is obtained. This enzyme concentration value is substituted into the reaction curve equation of 0.06 mmol/L glucose solution and the reaction absorbance value of 0.06 mmol/L glucose solution is obtained. The reaction absorbance values of 0.06 mmol/L glucose solution, 0.1 mmol/L glucose solution and 0.12 mmol/L glucose solution are taken as the vertical coordinates Y, and the corresponding substrate concentrations (0.06 mmol/L, 0.1 mmol/L, 0.12 mmol/L) are taken as the horizontal coordinates X. The standard curve for analyzing the substrate is obtained by fitting the linear equations.

By testing 0.1 mmol/L glucose solution, the absorbance Y is 0.5687. It is known that the curve equation of 0.1 mmol/L glucose is Y = 0.3692×ln(X)-0.0027, and the enzyme concentration X is calculated to be 4.7 U/mL.

Plug X= 4.7 into the upper and lower limit curve equations:
When the substrate is 0.12 mM, the absorbance is Y = 0.4208×ln4.7+0.0451, that is, 0.6963;
When the substrate is 0.06 mM, the absorbance is Y = 0.2529×ln4.7-0.0572, that is, 0.3342.

The standard curve for testing substrate is fitted when the enzyme concentration is 4.7 U/mL: the horizontal coordinates are substrate concentrations: 0.06, 0.10, 0.12 mmol/L, and the vertical coordinates are absorbances: 0.3342, 0.5687, 0.6963.

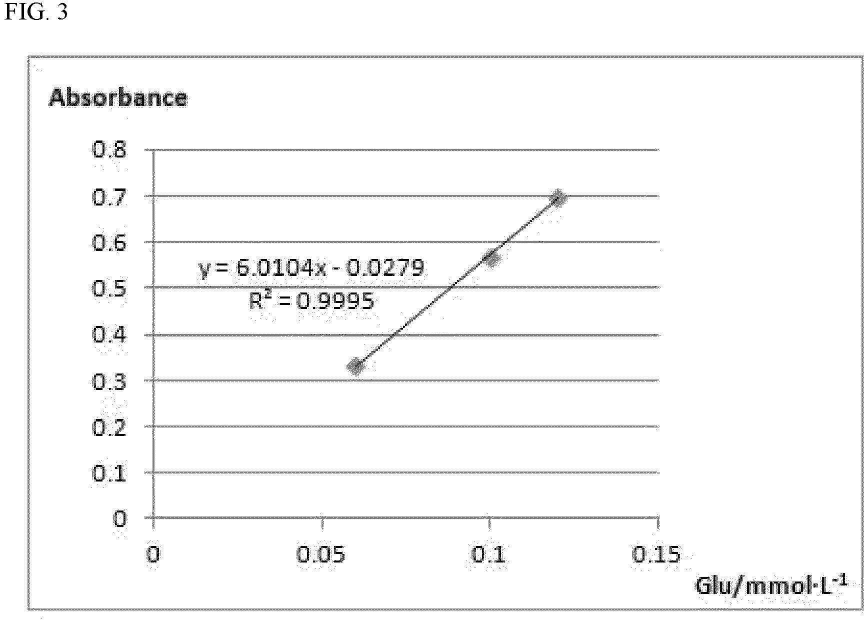

### Comparison with the Prior Art

The standard curve that uses 4.7 U/mL glucose oxidase to analyze 0.06-0.12 mmol /L glucose solution is created by the prior art, and the linear equation is y = 6.1365 x-0.0457, R<sup>2</sup>=0.9983.

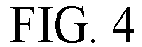

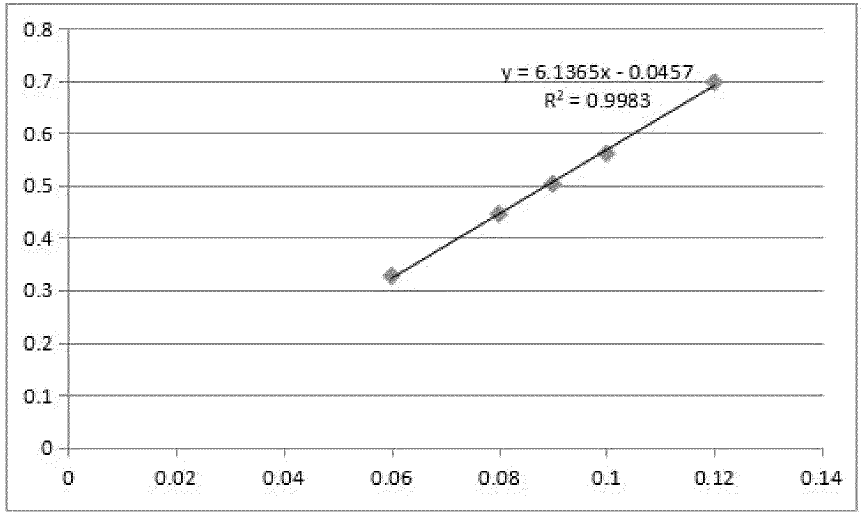

The glucose at same three concentrations are tested by the standard curve fitted by the prior art and the standard curve fitted by this invention. The relative deviations are calculated and the results, which do not exceed 5%.

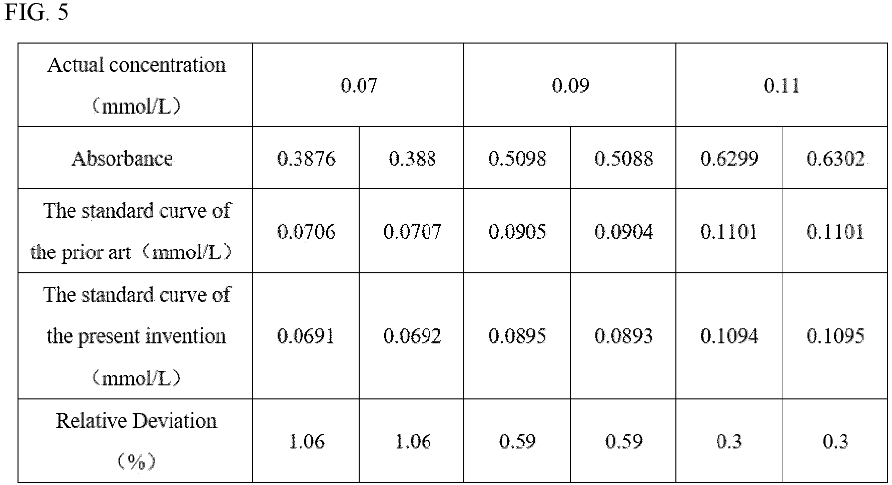

### Embodiment 2

The peroxidase-o-tolidine chromogenic reaction is employed, the standard curve is fitted and the concentration of galactose is analyzed. The galactose is catalyzed by the galactose oxidase to produce hydrogen peroxide. The horseradish peroxidase and o-tolidine are added and the reaction shows blue color. The absorbance is tested to analyze the galactose concentration. The range for analyzing the substrate is set to be 0.02 mmol/L - 0.12 mmol/L, and the suitable enzyme concentration range for testing is 7 U/mL - 11 U/mL.

Similarly, three curves of 0.02 mmol/L, 0.07 mmol/L and 0.12 mmol/L galactose solutions reacting separately with 7 U/mL, 8 U/mL, 9 U/mL, 10 U/mL and 11 U/mL galactose oxidase are created with the absorbances as the vertical coordinates and the enzyme concentrations as the horizontal coordinates.

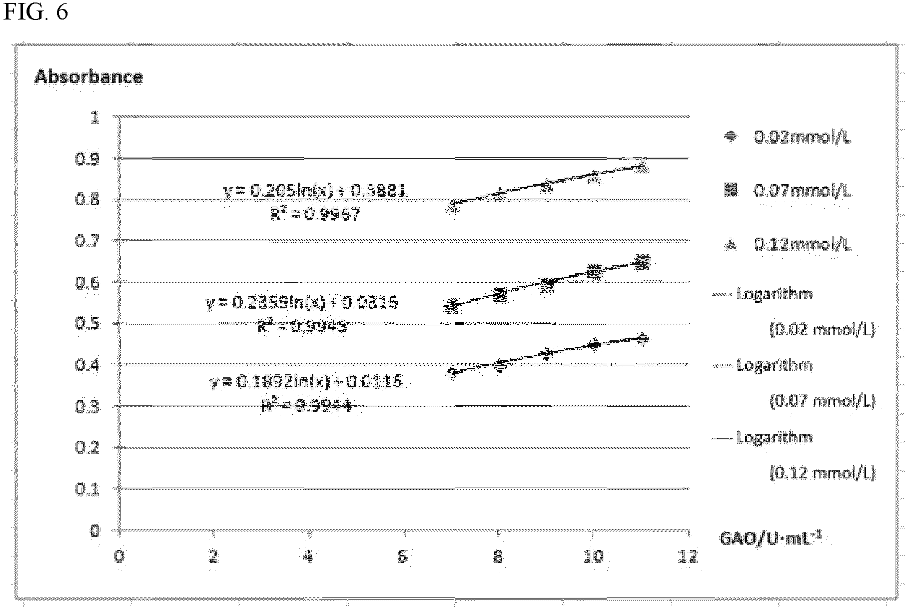

Any point of the enzyme in the range of 7 U/mL - 11 U/mL is used to react with 0.07 mmol/L galactose solution. The enzyme concentration value of this point can be calculated by substituting the reaction absorbance into the curve of 0.07 mmol/L galactose solution reacting with 7 U/mL - 11 U/mL enzyme concentrations. Simultaneously, the enzyme concentration value of this point is substituted into the reaction curve of 0.02 mmol/L and 0.12 mmol/L galactose solutions, and the absorbances of 0.02 mmol/L, 0.07 mmol/L and 0.12 mmol/L galactose solutions with the enzyme concentration of this point can be obtained. With these three absorbances as the vertical coordinates and the corresponding galactose concentrations as the horizontal coordinates, the standard curve for analyzing the galactose concentration is created.

The absorbance of 0.07 mmol/L galactose solution is tested to be 0.6077, and X=9.3 is calculated by the reaction equation of 0.07 mmol/L galactose solution Y=0.2359 × ln(X)+0.0816, that is, the enzyme concentration is 9.3 U/mL.

Plug X=9.3 into the two reaction equations of 0.02 mmol/L and 0.12 mmol/L:
When the substrate is 0.02 mmol/L, Y=0.1892× ln 9.3+0.0116, that is, the absorbance is 0.4335;
When the substrate is 0.12 mmol/L, Y=0.205×ln 9.3+0.3881, that is, the absorbance is 0.8453.

The curve for analyzing the substrate when the enzyme concentration is 9.3U/mL is fitted: the horizontal coordinates are 0.02, 0.07, 0.12 and the vertical coordinates are 0.4335, 0.6077, 0.8453.

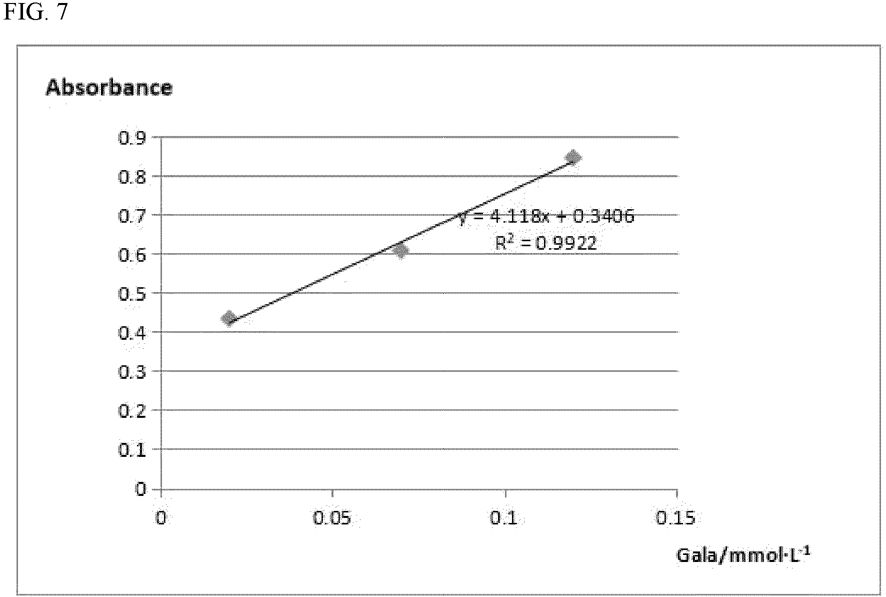

### Embodiment 3

The starch is catalyzed by the amylase to produce maltose, and, by utilizing the reducing properties of maltose, the brown 3-amino-5-nitrosalicylic acid is produced by the reaction of the maltose with 3, 5-dinitrosalicylic acid. The absorbance is measured, the standard curve is fitted, and the starch concentration is analyzed. The range for analyzing the substrate is set to be 0.3 mmol/L - 1.5 mmol/L, and the suitable enzyme concentration range for testing is 4 U/mL - 8 U/mL.

Three curves of 0.3 mmol/L, 0.9 mmol/L and 1.5 mmol/L starch reacting separately with 4 U/mL, 5 U/mL, 6 U/mL, 7 U/mL and 8 U/mL amylase are created.

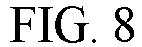

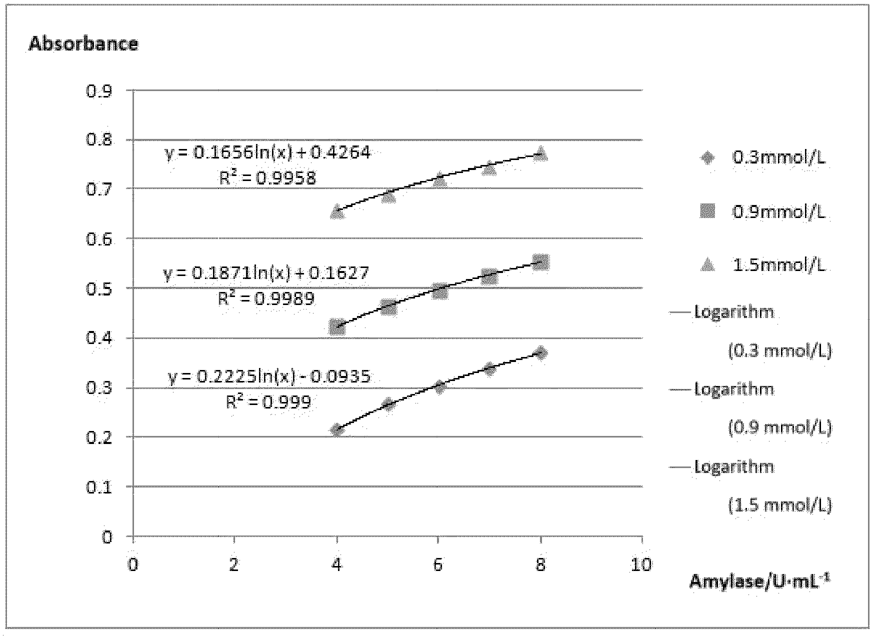

Any point of the enzyme in the range of 4 U/mL - 8 U/mL is used to react with 0.9 mmol/L starch. The enzyme concentration value of this point can be calculated by substituting the reaction value into the equation of 0.9 mmol/L starch reacting with the enzyme concentrations in the range of 4 U/mL - 8 U/mL. By substituting the enzyme concentration value of this point into the reaction equation of 0.3 mmol/L and 1.5 mmol/L starches separately, the reaction absorbance of 0.3 mmol/L, 0.9 mmol/L and 1.5 mmol/L starches with the enzyme concentration of this point can be obtained. Taking the three absorbance values as the vertical coordinates and the corresponding starch concentrations as the horizontal coordinates, the standard curve for analyzing starch concentration is created.

The reaction absorbance of 0.9 mmol/L starch is tested to be 0.5129, and X=6.5 is calculated by plugging the absorbance value into the 0.9 mmol/L reaction equation Y=0.1871 × ln(X)+0.1627, that is, the enzyme concentration of this point is 6.5 U/mL.

Plug X=6.5 into the reaction equation of 0.3 mmol/L and 1.5 mmol/L separately:
When the substrate is 0.3 mmol/L, Y=0.2225 × ln6.5-0.0935, that is, the absorbance is 0.3229;
When the substrate is 1.5 mmol/L, Y=0.1656 × ln6.5+0.4264, that is, the absorbance is 0.7364.

The standard curve for analyzing the substrate is fitted when the enzyme concentration is 6.5 U/mL: the horizontal coordinates are 0.3, 0.9, 1.5 and the vertical coordinates are 0.3229, 0.5129, 0.7364.

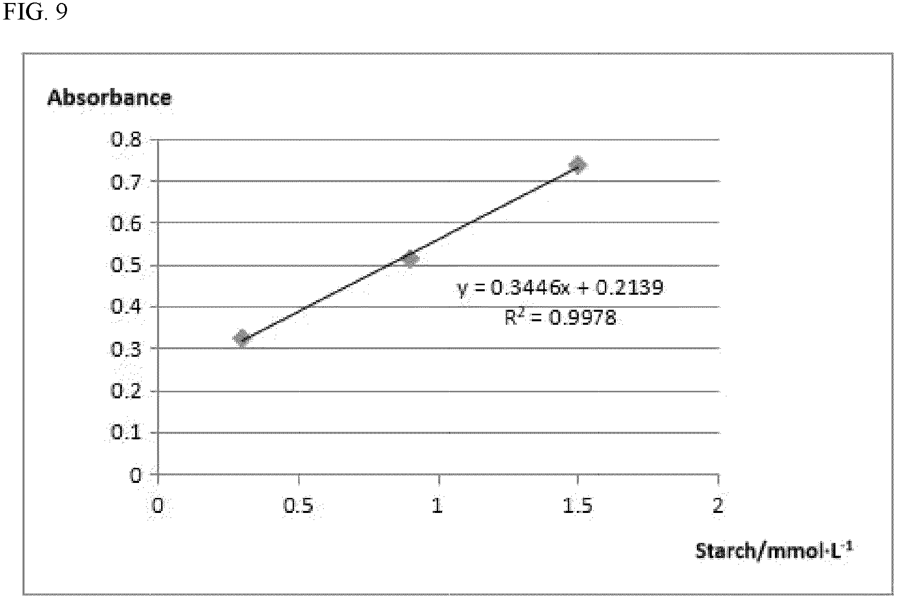

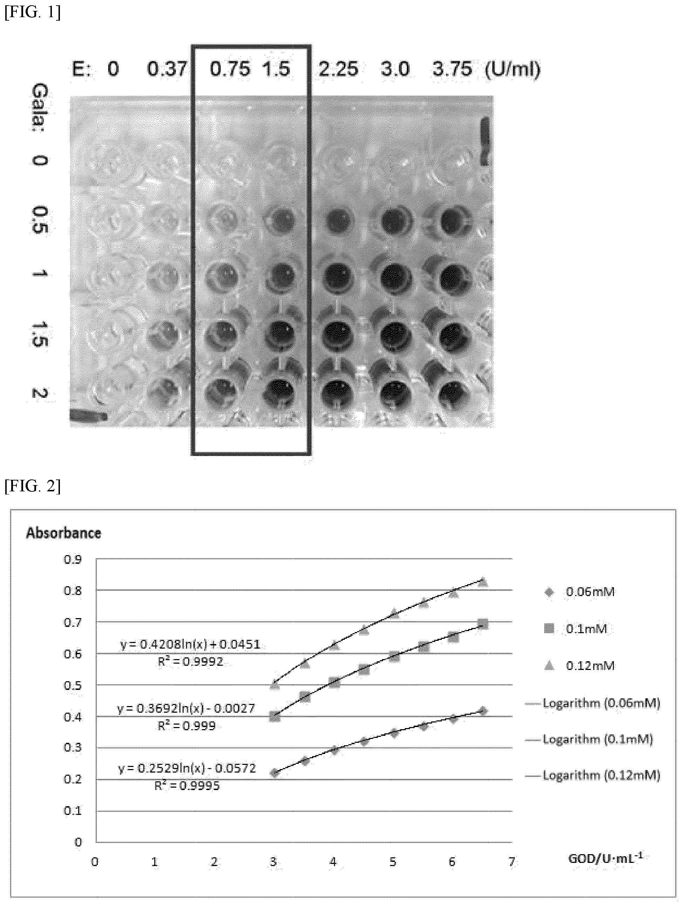

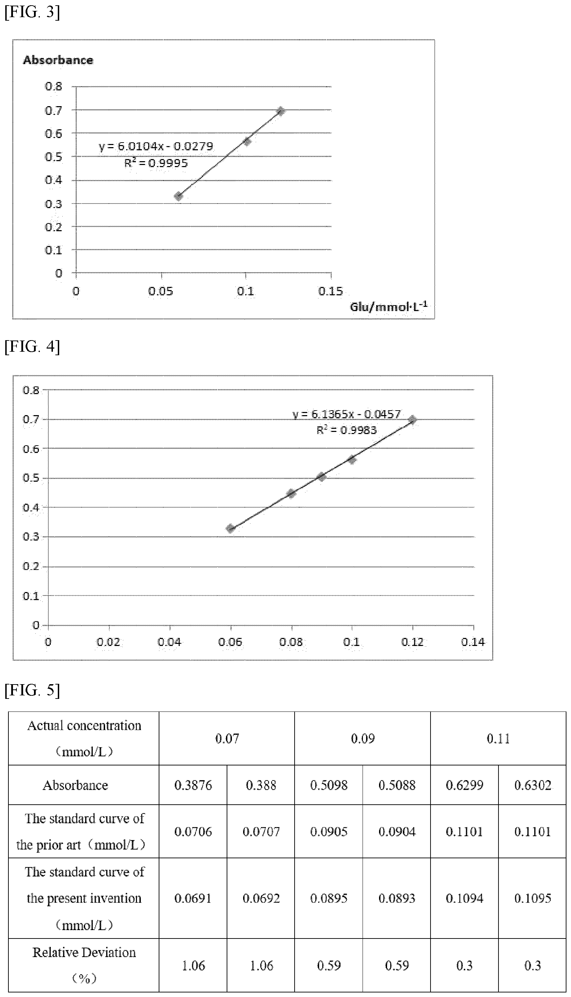

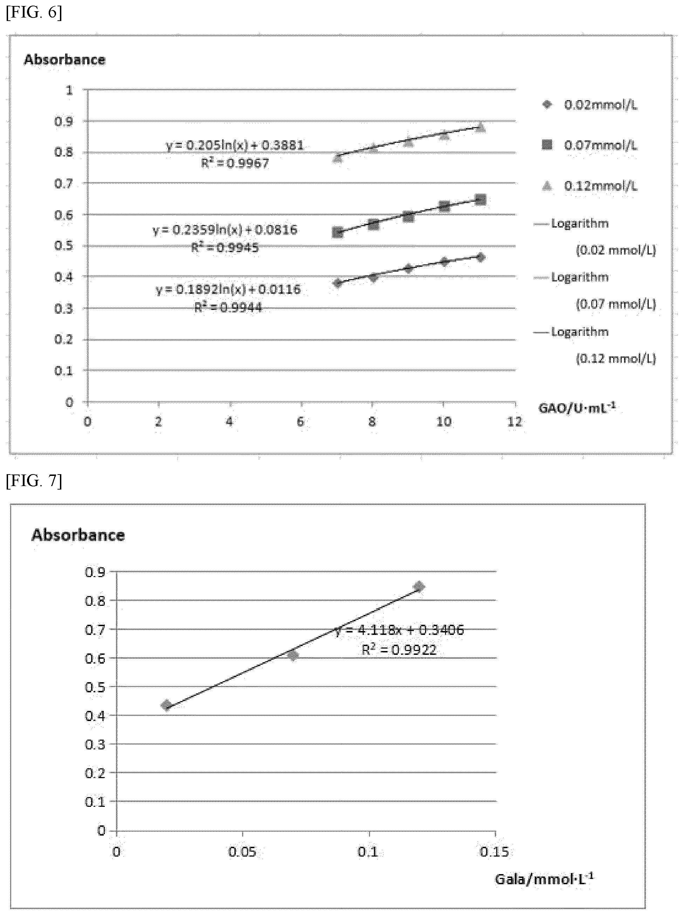

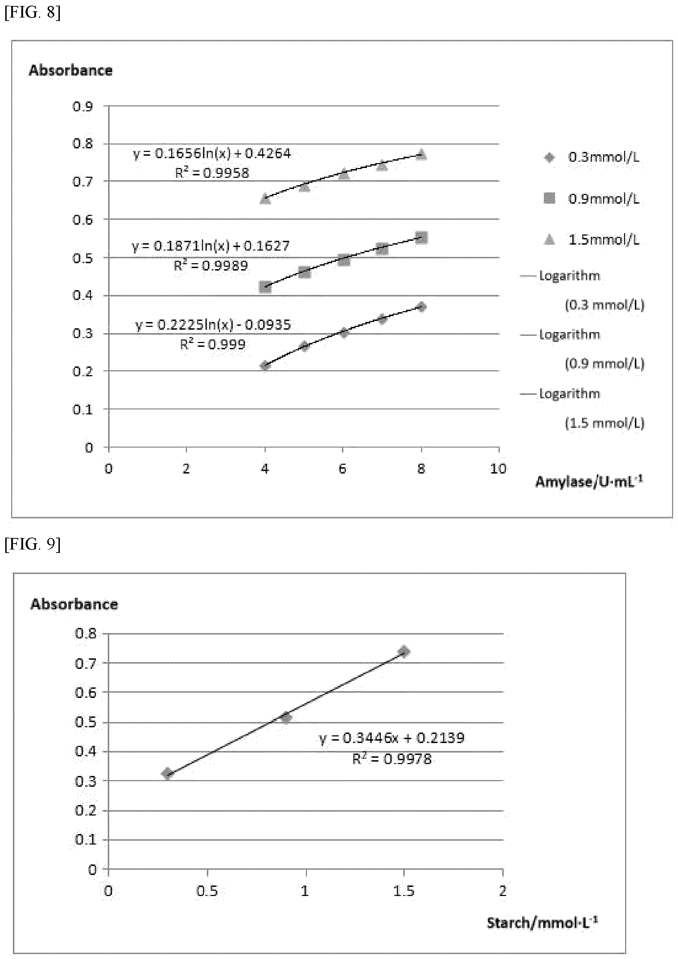

## Claims

1. A method of fitting the standard curve to analyze the substrate concentration by enzyme catalysis, wherein its characteristics feature the following steps: step 1, setting a range of concentrations for analyzing the substrate; step 2, testing optical signals by enzyme catalysis and determining a range of suitable tool enzyme concentrations for testing the substrate concentration in the range of step 1; step 3, by the enzyme catalysis process of step 2, selecting enzyme at multiple different concentrations within the range of the suitable tool enzyme concentrations set in step 2 to react with substrate at multiple different concentrations within the range of the substrate concentrations, and fitting at least three or more reaction curves and generating curve equations; step 4, using the reaction curves fitted in step 3 to test enzyme at any concentration within the range of the suitable tool enzyme concentrations in step 2 to obtain an optical signal; step 5, substituting the optical signal obtained in step 4 into the curve equation of the corresponding substrate in step 3 to obtain an enzyme concentration value; step 6, substituting the enzyme concentration value into the other curve equations in step 3 and obtaining three or more optical signals by calculation; step 7, taking three or more optical signals obtained in step 6 as vertical coordinates and corresponding substrate concentrations as horizontal coordinates, creating a curve and a curve equation, and obtaining the standard curve for testing the substrate concentration by enzyme at any concentration within the range of the suitable tool enzyme concentrations in step 2.

2. The method according to claim 1 of fitting the standard curve to analyze the substrate concentration by enzyme catalysis, wherein the optical signals refer to the visible light and the invisible light.

3. The method according to claim 1 of fitting the standard curve to analyze the substrate concentration by enzyme catalysis, wherein the enzyme catalysis is carried out in the same reaction system.

4. The method according to claim 1 of fitting the standard curve to analyze the substrate concentration by enzyme catalysis, wherein for the reaction curves fitted and the curve equations generated in step 3, the vertical coordinates are 3-5 or more optical signals obtained by the test and the horizontal coordinates are 3-5 or more corresponding tool enzyme concentrations.

5. The optical signals according to claim 2 refer to the visible light and the invisible light, wherein the visible light and the invisible light manifest as absorbance, reflection intensity, fluorescence intensity and chemiluminescence intensity.

6. The enzyme catalysis according to claim 3 is carried out under the same reaction system, wherein the same reaction system refers to setting the range of concentrations for analyzing the substrate and the range of the suitable tool enzyme concentrations, and other conditions in the reaction system remaining the same.

7. The curves and curve equations according to step 3 and step 7 of claim 1, wherein the curve is a directly proportional curve or an inversely proportional curve, and a curve having a determination coefficient R<sup>2</sup> close to 1 is selected.
